# EUROPÄISCHE PATENTSCHRIFT

(11) **EP 1 371 336 B1**
(45) Veröffentlichungstag und Bekanntmachung des Hinweises auf die Patenterteilung: **30.03.2005**
(21) Anmeldenummer: 03012098.4
(22) Anmeldetag: 28.05.2003
(51) Int. Cl.: A61B 17/22

(54) **Balg zur Ankopplung einer Quelle akustischer Wellen an ein Lebewesen**
Bellows for coupling a source of acoustic waves to a living being
Soufflet pour le couplage d'une source d'ondes acoustiques à un être vivant

(30) Priorität: 10.06.2002 DE 10225709
(43) Veröffentlichungstag der Anmeldung: 17.12.2003
(73) Patentinhaber: SIEMENS AKTIENGESELLSCHAFT, 80333 München (DE)
(72) Erfinder: Fehre, Jens, 91353 Hausen (DE); Granz, Bernd, Dr., 90522 Oberasbach (DE); Meinert, Christian, 91080 Marloffstein (DE); Nanke, Ralf, Dr., 91080 Spardorf (DE)

(56) Entgegenhaltungen:
- US-A- 4 674 505
- US-A- 4 928 672
- US-A1- 2001 046 184

## Beschreibung

Die Erfindung betrifft einen Balg zur Ankopplung einer ein akustisches Ausbreitungsmedium aufweisenden Quelle akustischer Wellen an ein Lebewesen.

Quellen akustischer Wellen werden beispielsweise in der Medizin zur Zertrümmerung von Konkrementen im Körperinneren eines Lebewesens oder zur Schmerztherapie eingesetzt. Eine derartige Quelle akustischer Wellen kann beispielsweise eine elektromagnetische Druckimpulsquelle, ein akustisches Ausbreitungsmedium, eine akustische Linse oder einen Hohlspiegel sowie einen Balg zur Ankopplung der Quelle akustischer Wellen an den Körper eines Lebewesens aufweisen. Mit der elektromagnetischen Druckimpulsquelle werden Druckimpulse in das akustische Ausbreitungsmedium eingeleitet, welche sich in dem akustischen Ausbreitungsmedium ausbreiten und durch die akustische Linse oder den Hohlspiegel fokussiert werden. Die Einleitung der fokussierten akustischen Wellen in den Körper des Lebewesens erfolgt über das akustische Ausbreitungsmedium und den Balg zur Ankopplung an das Lebewesen.

Mit einer Quelle akustischer Wellen erzeugte akustische Wellen werden derzeit nach dem Durchlaufen beispielsweise der akustischen Linse und vor dem Eintritt in den Körper des Lebewesens nicht mehr weiter geformt. Betrachtet man eine Fläche zwischen der akustischen Linse und der Körperoberfläche des Lebewesens, in welches die akustischen Wellen eingeleitet werden, senkrecht zur Hauptausbreitungsrichtung der akustischen Wellen, so stellt man fest, dass die akustischen Wellen bzw. das Feld akustischer Wellen innerhalb seiner -6db-Zone nicht homogen verteilt ist, sondern sich z. B. nahe der Mittelachse der Quelle akustischer Wellen Zonen sehr hohen positiven oder negativen Druckes befinden, die zu körperlichen Beeinträchtigungen des Lebewesens, insbesondere zu Irritationen der Haut des Lebewesens führen können.

In der DE 195 34 809 A1 ist eine Vorrichtung zur Behandlung von Körpergewebe und zur Zertrümmerung von Körpersteinen durch akustische Energie beschrieben. Um unerwünschte Abweichungen von der gradlinigen oder vorgesehenen Ausbreitung der akustischen Wellen beim Übergang von der Vorrichtung zu Körpergewebe zu vermeiden, wird unter anderem vorgeschlagen, die Geometrie der Grenzfläche so zu formen, dass Abweichungen nicht oder nur in kontrollierter Weise erfolgen. Beispielsweise lassen sich durch Andruck entsprechend geformter Applikatoren ebene, sphärische oder anderweitig kontrolliert geformte Grenzflächen erzeugen.

Aus der DE 33 12 014 A1 ist eine Einrichtung zur berührungslosen Zertrümmerung von Konkrementen im Körper von Lebewesen mit Hilfe von Stoßwellen bekannt. Die Stoßwellen werden dabei mit einem vorzugsweise elastisch verformbaren Kopplungskörper in den Körper eines Patienten eingekoppelt. Durch Verformung des Kopplungskörpers kann der Fokus der Stoßwellen in gewissen Grenzen auf das Behandlungsgebiet eingestellt werden. Zur Erweiterung der Einstellmöglichkeiten für den Fokus können zusätzlich Scheiben, welche vorzugsweise aus dem gleichen Material wie der Kopplungskörper sind, an den Kopplungskörper angefügt werden.

Der Erfindung liegt die Aufgabe zugrunde, einen Balg zur Ankopplung einer ein akustisches Ausbreitungsmedium aufweisenden Quelle akustischer Wellen an ein Lebewesen derart auszuführen, dass die körperliche Beeinträchtigung des Lebewesens bei der Einleitung von akustischen Wellen in den Körper des Lebewesens vermindert ist.

Nach der Erfindung wird diese Aufgabe gelöst durch einen Balg zur Ankopplung einer ein akustisches Ausbreitungsmedium aufweisenden Quelle akustischer Wellen an ein Lebewesen, wobei der Balg in dem Bereich, in dem er zur Einleitung von akustischen Wellen in das Lebewesen an dem Lebewesen zur Anlage gebracht werden kann, ein auf der dem Lebewesen abgewandten Seite des Balges angeordnetes, mit dem Balg fest verbundenes geometrisches Element aufweist, welches in dem Ausbreitungsweg von mit der Quelle akustischer Wellen erzeugten akustischen Wellen liegt und die akustischen Wellen gezielt formt. Erfindungsgemäß ist es also vorgesehen, einen modifizierten Balg an einer Quelle akustischer Wellen einzusetzen, um die mit der Quelle akustischer Wellen erzeugten Wellen nach einer ersten Formung durch eine akustische Linse oder einen Hohlspiegel nochmals gezielt beeinflussen, führen bzw. formen zu können, wobei durch den Ausgleich von Inhomogenitäten des Schallfeldes körperliche Beeinträchtigungen des mit den akustischen Wellen zu behandelnden Lebewesens, insbesondere der Haut des Lebewesens, zumindest reduziert werden. Dadurch dass sich das geometrische Element auf der dem Lebewesen abgewandten Seite des Balges befindet, wird eine gute, für den Patienten angenehme Anlage des Balges an der Körperoberfläche gewährleisten.

Nach einer Ausführungsform der Erfindung handelt es sich bei dem geometrischen Element um einen akustischen Spiegel. Der akustische Spiegel kann plan oder kegelförmig ausgebildet sein oder wenigstens zwei geneigte Spiegelflächen aufweisen, so dass die erzeugten akustischen Wellen bzw. ein Feld aus akustischen Wellen bei Durchtritt durch den akustischen Spiegel durch teilweise Reflexionen geformt wird.

Eine andere Formung eines Feldes von akustischen Wellen lässt sich nach einer Variante der Erfindung dadurch erreichen, dass das auf dem Balg angeordnete geometrische Element eine Vielzahl von regelmäßig oder unregelmäßig angeordneten Objekten aufweist. Dadurch wird eine Zerstreuung eines Feldes erzeugter akustischer Wellen bewirkt, wodurch ein Feld akustischer Wellen ebenfalls geformt werden kann. Bei den Objekten kann es sich beispielsweise um ein Feld aus kleinen Kegeln oder auch um ein Feld aus geometrisch anders geformten Objekten handeln, deren größte Ausdehnung klein zur Wellenlänge der mit der Quelle akustischer Wellen erzeugten akustischen Wellen ist. Die größte Ausdehnung der Objekte in einer Richtung sollte dabei ca. einen Größenfaktor, also um das Zehnfache, kleiner sein als die Wellenlänge der mit der Quelle akustischer Wellen erzeugten akustischen Wellen.

Nach Varianten der Erfindung lässt sich ein Feld akustischer Wellen auch durch ein als akustisches Filter wirkendes, auf dem Balg angeordnetes Element formen, wobei das akustische Filter ein Hochpass- oder ein Tiefpassfilter für die akustischen Wellen sein kann. Das Element ist in der Regel derart ausgebildet, dass es mehrere Schichten mit Filterwirkung umfasst.

Andere Varianten der Erfindung sehen vor, dass es sich bei dem auf dem Balg angeordneten Element um eine akustische Linse oder einen Absorber für akustische Wellen handelt, wodurch ein mit der Quelle akustischer Wellen erzeugtes Feld akustischer Wellen ebenfalls gezielt geformt werden kann, so dass insbesondere an der Hautoberfläche eines Lebewesens auftretende Irritationen infolge der in den Körper des Lebewesens eingeleiteten akustischen Wellen zumindest reduziert werden können.

Nach einer Ausführungsform der Erfindung ist das auf dem Balg angeordnete geometrische Element aus dem gleichen Material ausgebildet wie der Balg zur Ankopplung selbst. Die Eigenschaft des Elementes als Spiegel, als Zerstreuer, als Filter, als Linse oder als Absorber für akustische Wellen ergibt sich unter anderem dabei durch den Aufbau bzw. durch die geometrische Formung des Elements.

Nach einer anderen Ausführungsform der Erfindung ist das geometrische Element aus einem anderen Material als der Balg ausgebildet, welches sich bezüglich seiner akustischen Eigenschaften vorzugsweise deutlich von den akustischen Eigenschaften des akustischen Ausbreitungsmediums unterscheidet. Geeignete Materialien für die Ausbildung des Elementes sind gummiartige Materialien sowie Hart-PVC, metallhaltige Materialien sowie generell alle Materialien, welche sich gut mit dem Material des Balges verbinden lassen, welcher in der Regel aus Weich-PVC, Latex oder Silikon ausgebildet ist.

Die vorliegende Aufgabe wird auch gelöst durch einen Balg zur Ankopplung einer ein akustisches Ausbreitungsmedium aufweisenden Quelle akustischer Wellen an ein Lebewesen, wobei der Balg in dem Bereich, in dem er zur Einleitung von akustischen Wellen in das Lebewesen an dem Lebewesen zur Anlage gebracht werden kann, eine auf der dem Lebewesen abgewandten Seite des Balges definiert geometrisch veränderte Zone aufweist, welche in dem Ausbreitungsweg von mit der Quelle akustischer Wellen erzeugten akustischen Wellen liegt und die akustischen Wellen gezielt formt. Bei dieser geometrischen Modifikation des Balges handelt es sich also um eine definiert geometrisch veränderte Zone des Balges selbst.

Gemäß Varianten der Erfindung kann die Zone des Balges derart ausgebildet sein, dass sie die Wirkung eines akustischen Spiegels entfaltet. Die Zone kann in Form eines planen oder kegelförmigen akustischen Spiegels oder in Form eines wenigstens zwei geneigte Spiegelflächen aufweisenden akustischen Spiegels ausgebildet sein.

Nach einer Ausführungsform der Erfindung weist die Zone des Balges eine Vielzahl von regelmäßig oder unregelmäßig angeordneten Objekten auf, so dass die Zone des Balges eine zerstreuende Wirkung für akustische Wellen entfaltet. Wie bereits für das Element beschrieben, können die Objekte kleine Kegel oder geometrisch anders geformte Objekte sein, welche klein, d. h. um wenigstens einen Größenfaktor in ihrer größten Ausdehnung kleiner als die Wellenlänge der mit der Quelle akustischer Wellen erzeugten akustischen Wellen sind.

Nach weiteren Ausführungsformen der Erfindung kann die Zone des Balges derart ausgebildet sein, dass sie die Wirkung eines akustischen Filters, vorzugsweise die Wirkung eines akustischen Hochpass- oder eines akustischen Tiefpassfilters, entfaltet.

Andere Varianten der Erfindung sehen vor, dass die Zone des Balges derart ausgebildet ist, dass sie die Wirkung einer akustischen Linse oder die Wirkung eines Absorbers für akustische Wellen entfaltet.

Außerdem wird die vorliegende Aufgabe gelöst durch einen Balg zur Ankopplung einer ein akustisches Ausbreitungsmedium aufweisenden Quelle akustischer Wellen an ein Lebewesen, wobei der Balg in dem Bereich, in dem er zur Einleitung von akustischen Wellen in das Lebewesen an dem Lebewesen zur Anlage gebracht werden kann, einen Wandabschnitt aufweist, welcher aus einem anderen Material als der Rest des Balges ausgebildet ist, welcher Wandabschnitt in dem Ausbreitungsweg von mit der Quelle akustischer Wellen erzeugten akustischen Wellen liegt und die akustischen Wellen gezielt formt.

Ausführungsbeispiele der Erfindung sind in den beigefügten schematischen Zeichnungen dargestellt. Es zeigen:
- Fig. 1 bis 12: verschiedene Ausführungsformen eines eine geometrische Modifikation aufweisenden Balges zur Ankopplung einer ein akustisches Ausbreitungsmedium aufweisenden Quelle akustischer Wellen an ein Lebewesen und
- Fig. 13, 14: Balge mit jeweils einem aus einem anderen Material als der Balg ausgebildeten Abschnitt.

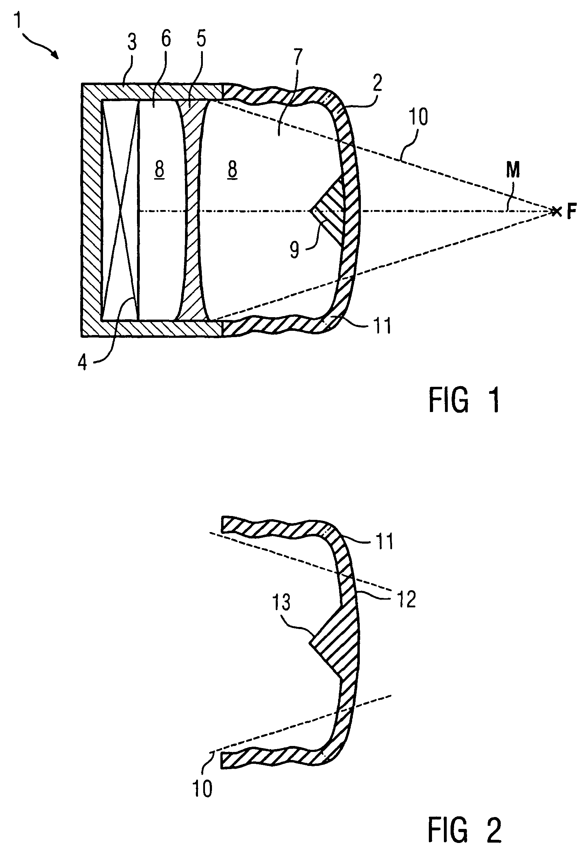

Die Fig. 1 zeigt in schematischer, teils geschnittener Darstellung einen Therapiekopf 1 zur Erzeugung akustischer Wellen, welcher beispielsweise Teil eines Lithotripters sein kann. Der Therapiekopf 1 weist ein patientenseitig, mit einem Ankoppelbalg 2 verschlossenes Gehäuse 3 auf, in dem im Falle des vorliegenden Ausführungsbeispieles eine elektromagnetische Druckimpulsquelle 4, eine fokussierende Linse 5 und in den beiden Räumen 6 und 7 ein akustisches Ausbreitungsmedium 8 enthalten sind. Mit dem Therapiekopf 1 können in an sich bekannter Weise akustische Wellen, insbesondere Stoßwellen, erzeugt werden, wobei mittels der akustischen Druckimpulsquelle 4 Druckimpulse in das akustische Ausbreitungsmedium 8 eingeleitet werden, welche sich während ihrer Ausbreitung zu Stoßwellen aufsteilen, mittels der fokussierenden Linse 5 auf eine, wenigstens im Wesentlichen auf der Mittelachse M des Therapiekopfes 1 liegende Fokuszone F fokussiert werden und mit dem Ankoppelbalg 2 in einen nicht dargestellten Körper eines Patienten, an dem der Ankoppelbalg 2 zur Anlage gebracht worden ist, eingeleitet werden können.

Wie bereits eingangs erwähnt, können bei der Einleitung von Stoßwellen in den Körper eines Patienten aufgrund der Inhomogenität des erzeugten Stoßwellenfeldes innerhalb seiner -6db-Zone Beeinträchtigungen des Patienten, insbesondere Irritationen der Haut des Patienten, auftreten. Um diesen gezielt entgegenzuwirken, ist der Ankoppelbalg 2 im Falle des in Fig. 1 gezeigten Ausführungsbeispiels derart ausgebildet, dass er in dem mit strichpunktierten Linien angedeuteten Bereich 11, in dem der Ankoppelbalg 2 zur Einleitung der Stoßwellen in den Patienten an dem Patienten zur Anlage gebracht werden kann, ein kegelförmiges Element 9 aufweist, welches auf der dem Patienten abgewandten Seite des Ankoppelbalges 2 angeordnet ist. Das kegelförmige Element 9 entfaltet die Wirkung eines akustischen Spiegels. Wie der Fig. 1 entnommen werden kann, liegt der akustische Spiegel 9 in dem mit strichlierten Linien angedeuteten Ausbreitungsweg 10 der a-kustischen Wellen. Mit Hilfe des akustischen Spiegels 9 können die erzeugten und sich in dem akustischen Ausbreitungsmedium 8 fortpflanzenden akustischen Wellen nach der Formung durch die fokussierende Linse 5 vor Einleitung in den Körper des Patienten nochmals durch teilweise Reflexionen an dem akustischen Spiegel 9 geformt werden, so dass Beeinträchtigungen des Patienten, insbesondere Irritationen der Haut des Patienten, durch die akustischen Wellen vermindert werden.

In den Fig. 2 bis 12 sind weitere Ankoppelbalge dargestellt, welche anstelle des in der Fig. 1 dargestellten Ankoppelbalges 2 an dem Gehäuse 3 des Therapiekopfes 1 angeordnet sein können und das Gehäuse des Therapiekopfes 1 wie der Ankoppelbalg 2 patientenseitig abschließen. Die Ankoppelbalge sind also gegeneinander austauschbar.

Der in Fig. 2 gezeigte Ankoppelbalg 12 unterscheidet sich von dem in Fig. 1 gezeigten Ankoppelbalg 2 dadurch, dass kein separat ausgeführtes Element 9 auf dem Ankoppelbalg 12 angeordnet ist, sondern dass der Ankoppelbalg 12 selbst auf seiner dem Patienten abgewandten Seite eine geometrisch veränderte Zone 13 aufweist, welche kegelförmig ausgebildet ist und die Funktion eines akustischen Spiegels entfaltet. Die geometrisch veränderte Zone 13 des Ankoppelbalges 12 befindet sich in dem mit strichpunktierten Linien angedeuteten Bereich 11 des Ankoppelbalges 12, der zur Einleitung von akustischen Wellen in den Körper eines Patienten an dem Patienten zur Anlage gebracht werden kann. Außerdem befindet sich die Zone 13 im Ausbreitungsweg 10 von mit dem Therapiekopf 1 erzeugten akustischer Wellen, so das erzeugte und sich ausbreitende akustische Wellen vor dem Eintritt in der Körper eines Patienten nochmals gezielt geformt werden können.

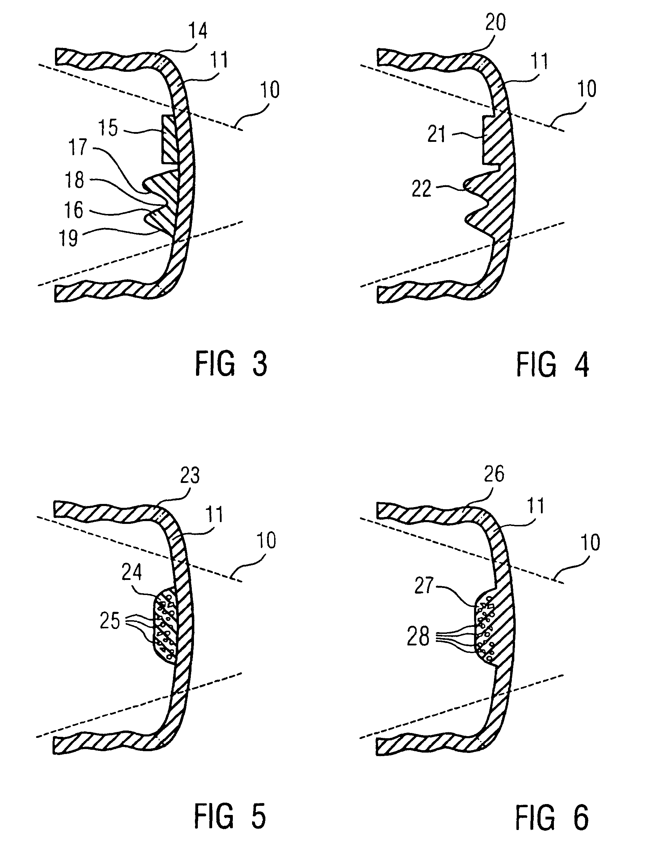

In Fig. 3 ist ein Ankoppelbalg 14 gezeigt, welcher zwei Elemente 15 und 16 aufweist, wobei das Element 15 ein plan ausgebildeter akustischer Spiegel und das Element 16 ein akustischer Spiegel mit drei geneigten Spiegelflächen 17, 18 und 19 ist. Auch mit den auf der dem Patienten abgewandten Seite des Ankoppelbalges 14 angeordneten akustischen Spiegeln 15 und 16 kann das Stoßwellenfeld nach Formung durch die fokussierende Linse 5 vor Eintritt in den Körper des Patienten nochmals geformt werden, um Beeinträchtigungen des Lebewesens durch die Stoßwellen zu vermeiden. Der in Fig. 4 gezeigte Ankoppelbalg 20 unterscheidet sich von dem in Fig. 3 gezeigten Ankoppelbalg 14 dahingehend, dass der plane, dem akustischen Spiegel 15 entsprechende akustische Spiegel 21 und der drei geneigte Spiegelflächen aufweisende, dem akustischen Spiegel 16 entsprechende akustische Spiegel 22 definiert geometrisch veränderte Zonen des Ankoppelbalges 20 selbst sind, also nicht wie die akustischen Spiegel 15 und 16 separat ausgeführte, auf dem Ankoppelbalg angeordnete Elemente sind.

In Fig. 5 ist ein Ankoppelbalg 23 dargestellt, welcher auf seiner dem Patienten abgewandten Seite ein Element 24 mit einer Vielzahl von regelmäßig oder unregelmäßig angeordneten Objekten 25 aufweist. Bei den Objekten 25 kann es sich um kleine Kegel oder andere geometrische Objekte handeln, welche in ihrer größten Ausdehnung klein zur Wellenlänge der mit dem Therapiekopf 1 erzeugten akustischen Wellen sind. Vorzugsweise ist die größte Ausdehnung der Objekte 25 um wenigstens einen Größenfaktor kleiner als die Wellenlänge der erzeugten akustischen Wellen. Die Objekte 25 des Elementes 24 haben zerstreuende Wirkung auf akustische Wellen. Infolge der Anordnung des Elementes 24 im Ausbreitungsweg 10 der akustischen Wellen sowie in dem Bereich 11 des Ankoppelbalges 23, welcher zur Einleitung der akustischen Wellen in einen Patienten an dem Patienten zur Anlage gebracht werden kann, hat das Element 24 also die Funktion eines Zerstreuers auf akustische Wellen und formt somit das Feld der erzeugten akustischen Wellen vor dem Eintritt in den Körper des Patienten nochmals. Der in Fig. 6 gezeigte Ankoppelbalg 26 unterscheidet sich von dem in Fig. 5 gezeigten Ankoppelbalg 23 dadurch, dass er selbst eine definiert geometrisch veränderte Zone 27 aufweist, welche eine Vielzahl von regelmäßig oder unregelmäßig angeordneten Objekten 28 aufweist, die in die Zone 27 eingebracht wurden. Bei den Objekten 28 kann es sich wiederum um kleine Kegel oder anders geformte geometrische Objekte handeln. Die mit den Objekten 28 versehene Zone 27 des Ankoppelbalges 26 wirkt wie das Element 24 des Ankoppelbalges 23 zerstreuend auf akustische Wellen.

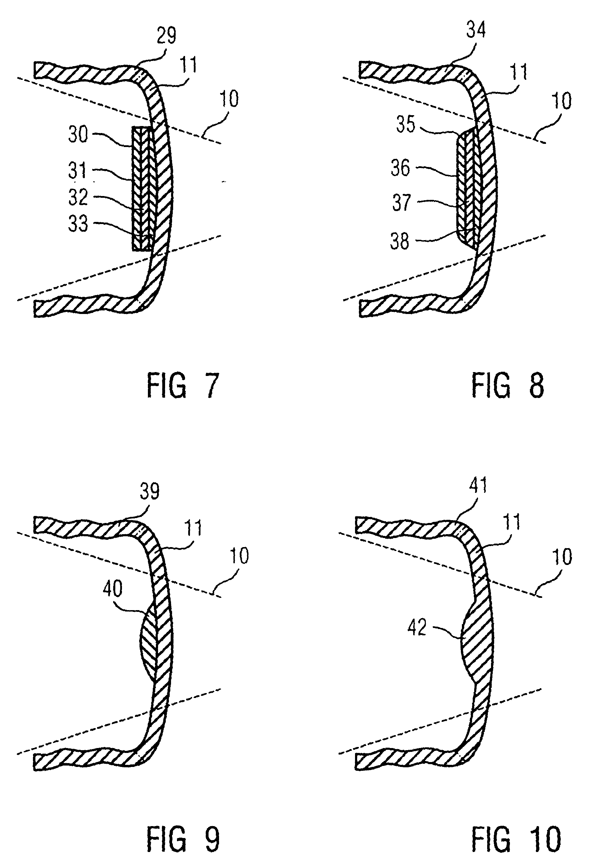

Der in Fig. 7 gezeigte Ankoppelbalg 29 weist auf seiner dem Patienten abgewandten Seite ein Element 30 auf, bei dem es sich im Falle des vorliegenden Ausführungsbeispieles um ein Feld aus drei Filterschichten 31, 32 und 33 handelt. Auch mit Hilfe des Feldes der Filterschichten 31, 32 und 33, bei denen es sich um Tiefpass- oder um Hochpassfilter für akustische Wellen handeln kann, kann das Feld von akustischen Wellen vor dem Eintritt der akustischen Wellen in den Körper des Patienten nochmals geformt werden, in dem nur akustische Wellen einer bestimmten Frequenz in den Körper des Patienten gelangen können. Der in Fig. 8 gezeigte Ankoppelbalg 34 unterscheidet sich von dem in Fig. 7 gezeigten Ankoppelbalg 29 wieder dadurch, dass der Ankoppelbalg 34 selbst in dem Bereich 11, in dem er zur Einleitung von akustischen Wellen in den Patienten an dem Patienten zur Anlage gebracht werden kann, und welcher Bereich 11 in dem Ausbreitungsweg 10 der akustischen Wellen liegt, derart definiert geometrisch geformt ist, dass er die Funktion dreier akustischer Frequenzfilter 36, 37 und 38 entfaltet.

In Fig. 9 ist ein weiterer Ankoppelbalg 39 gezeigt, auf dessen dem Patienten abgewandter Seite ein Element 40 angeordnet ist, welches die Wirkung einer Linse für akustische Wellen entfaltet. Bei der Linse kann es sich um eine fokussierende oder auch um eine defokussierende Linse handeln, welche das Feld der mit dem Therapiekopf 1 erzeugten akustischen Wellen vor dem Eintritt in den Körper des Patienten nochmals formen kann. Der in Fig. 10 dargestellte Koppelbalg 41 unterscheidet sich von dem in Fig. 9 dargestellten Koppelbalg 39 wiederum dahingehend, dass er selbst eine definiert geometrisch veränderte Zone 42 aufweist, welche die Funktion einer akustischen Linse entfaltet. Die Funktion einer akustischen Linse kann beispielsweise, wie in den Fig. 9 und 10 gezeigt, dadurch erreicht werden, dass das an den Ankoppelbalg 39 angeordnete Element 40 bzw. die Zone 42 des Ankoppelbalgs 41 eine konvexe Krümmung aufweisen.

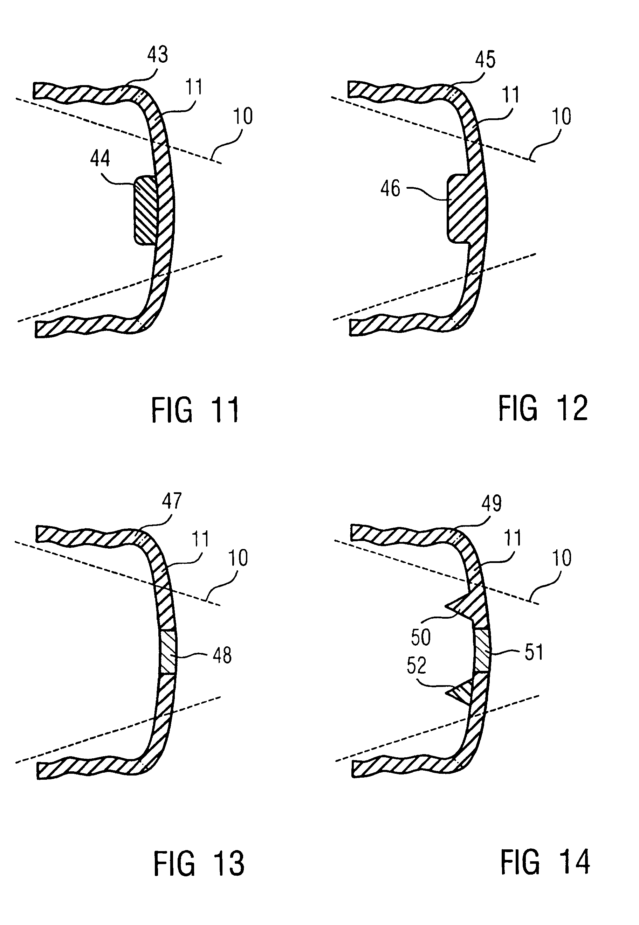

In Fig. 11 ist ein weiterer Ankoppelbalg 43 dargestellt, auf dessen dem Patienten abgewandter Seite ein Element 44 angeordnet ist, welches die Wirkung eines Absorbers für akustische Wellen entfaltet. Der in Fig. 12 dargestellte Ankoppelbalg 45 weist im Unterschied zu dem in Fig. 11 dargestellten Ankoppelbalg 43 selbst eine definiert geometrisch veränderte Zone 46 auf, welche die Wirkung eines Absorbers für akustische Wellen entfaltet.

Bei den in den Fig. 1, 3, 5, 7, 9 und 11 dargestellten Ausführungsbeispielen können die jeweiligen Elemente der Ankoppelbalge aus dem gleichen Material ausgebildet sein wie der Ankoppelbalg selbst. In den in den Fig. 2, 4, 6, 8, 10 und 12 dargestellten Ausführungsbeispielen von Ankoppelbalgen sind die Zonen der Ankoppelbalge ohnehin aus dem gleichen Material wie der Ankoppelbalg ausgebildet. Bei den in den Fig. 1, 3, 5, 7 und 9 dargestellten Ankoppelbalgen können die Elemente aber auch aus einem anderen Material als der Ankoppelbalg, welches sich bezüglich seiner akustischen Eigenschaften von den Eigenschaften des akustischen Ausbreitungsmediums vorzugsweise deutlich unterscheidet, ausgebildet sein. Ankoppelbalge sind in der Regel aus Weich-PVC, Latex oder Silikon ausgebildet. Geeignete Materialien für die Ausbildung der in den Fig. 1, 3, 5, 7 und 9 dargestellten Elemente sind dabei ebenfalls gummiartige Materialien, beispielsweise Hartgummi, Hart-PVC sowie alle anderen Materialien, welche sich gut mit dem Material des jeweiligen Ankoppelbalges verbinden lassen. Die Elemente der in den Fig. 1, 3, 5, 7, 9 und 11 gezeigten Ankoppelbalge können beispielsweise auf die Ankoppelbalge geklebt oder in anderer Weise fest mit den Ankoppelbalgen verbunden sein. Die Objekte 25 und 28 der Ankoppelbalge 23 und 26 können beispielsweise aus Metall sein.

In Fig. 13 ist ein weiterer erfindungsgemäßer Ankoppelbalg 47 dargestellt, welcher sich von den bisher beschriebenen Ankoppelbalgen dadurch unterscheidet, dass er in dem Bereich 11, in dem er zur Einleitung von akustischen Wellen in den Patienten an dem Patienten zur Anlage gebracht werden kann, einen Wandabschnitt 48 aufweist, welcher aus einem anderen Material als der Rest des Ankoppelbalges 47 ausgebildet ist. Bei dem Material des Wandabschnittes 47 kann es sich ebenfalls um ein gummiartiges Material, wie Hartgummi, Hart-PVC sowie um alle Materialien handeln, welche sich gut mit dem Material des Ankoppelbalges verbinden lassen, welcher, wie bereits erwähnt, in der Regel aus Weich-PVC, Latex oder Silikon ausgebildet ist. Demnach lässt sich auch auf diese Weise in Abhängigkeit von den akustischen Eigenschaften des Materials des Wandabschnittes 48 ein Feld akustischer Wellen vor Eintritt in den Körper eines Patienten nochmals formen.

Im Übrigen sind Mischformen zwischen den beschriebenen Ankoppelbalgen möglich. So kann ein Koppelbalg, welcher selbst eine oder mehrere geometrisch veränderte Zonen zur Formung eines Feldes akustischer Wellen aufweist, zusätzlich mit einem oder mehreren Elementen versehen sein, wobei eine Zone bzw. ein Element als akustischer Spiegel, Filter, Zerstreuer, Absorber oder Linse wirken kann. Darüber hinaus kann ein derartiger Ankoppelbalg zusätzlich einen Abschnitt aufweisen, welcher aus einem anderen Material als der Rest des Ankoppelbalges ausgebildet ist. In Fig. 14 ist eine solche Mischform gezeigt. Der Ankoppelbalg 49 weist eine kegelförmig ausgebildete als akustischer Spiegel wirkende Zone 50, einen aus einem anderen Material als der Rest des Ankoppelbalges 49 ausgebildeten Abschnitt 51 und ein auf dem Ankoppelbalg 49 angeordnetes kegelförmiges Element 52 auf, welches ebenfalls als akustischer Spiegel wirkt.

Die in den Fig. 1 bis 14 dargestellten Ankoppelbalge lassen sich alternativ an dem in Fig. 1 gezeigten Therapiekopf 1 einsetzen. Die Ankoppelbalge sind in der Regel leicht auszuwechseln und preisgünstig herstellbar, so dass durch die vorliegende Erfindung die Möglichkeit geschaffen ist, das Stoßwellenfeld eines Therapiekopfes 1 durch gezielten Austausch von Ankoppelbalgen den verschiedenen Anforderungen in der Therapie anzupassen und somit eine höhere Schonung des zu behandelnden Lebewesens zu erreichen. Da die jeweils an dem Ankoppelbalg getroffene Maßnahme, sei es durch Anordnung eines speziell geformten Elementes oder durch eine Formung des Koppelbalges selbst oder durch einen Abschnitt aus einem anderen Material, etwa flächenanteilig in die Energie der Stoßwelle in der Fokuszone F eingeht, ist die Änderung der Energie in der Fokuszone berechenbar und die Fokuszone demnach auch gezielt formbar. Die an dem Ankoppelbalg getroffene Maßnahme verändert die Fokuszone und die Desintegrationswirkung der Stoßwellen in der Regel aber nur gering. Welcher der exemplarisch beschriebenen Ankoppelbalge an einem bestimmten Therapiekopf zur Formung eines Feldes akustischer Wellen eingesetzt wird, hängt einerseits von der vorzunehmenden Therapie und andererseits von den messtechnisch ermittelbaren Eigenschaften des bestimmten Therapiekopfes im Hinblick auf die Homogenität des mit dem Therapiekopfes erzeugbaren Feldes von akustischen Wellen ab.

Die Erfindung wurde vorstehend am Beispiel eines Therapiekopfes mit einer elektromagnetischen Druckimpulsquelle beschrieben. Der Therapiekopf muss jedoch nicht notwendigerweise eine elektromagnetische Druckimpulsquelle, sondern kann auch eine andere, beispielsweise eine piezoelektrische Druckimpulsquelle aufweisen.

Des Weiteren muss nicht notwendigerweise eine Formung des Stoßwellenfeldes durch eine fokussierende Linse vor einer Formung des Stoßwellenfeldes durch das Element des Ankoppelbalges oder die geometrisch veränderte Zone des Ankoppelbalges oder den Abschnitt anderen Materials des Ankoppelbalges stattgefunden haben.

## Patentansprüche

1. Balg zur Ankopplung einer ein akustisches Ausbreitungsmedium (8) aufweisenden Quelle akustischer Wellen (1) an ein Lebewesen, wobei der Balg (2, 14, 23, 29, 39, 43) in dem Bereich (11), in dem er zur Einleitung von akustischen Wellen in das Lebewesen an dem Lebewesen zur Anlage gebracht werden kann, ein auf der dem Lebewesen abgewandten Seite des Balges (2, 14, 23, 29, 39, 43) angeordnetes, mit dem Balg (2, 14, 23, 29, 39, 43) fest verbundenes geometrisches Element (9, 15, 16, 24, 30, 40, 44) aufweist, welches in dem Ausbreitungsweg (10) von mit der Quelle akustischer Wellen (1) erzeugten akustischen Wellen liegt und die akustischen Wellen gezielt formt.

2. Balg nach Anspruch 1, bei dem das Element (9, 15, 16) ein akustischer Spiegel ist.

3. Balg nach Anspruch 2, bei dem der akustische Spiegel (9, 15) plan oder kegelförmig ausgebildet ist.

4. Balg nach Anspruch 2, bei dem der akustische Spiegel (16) wenigstens zwei geneigte Spiegelflächen (17, 18, 19) aufweist.

5. Balg nach Anspruch 1, bei dem das Element (24) eine Vielzahl von regelmäßig oder unregelmäßig angeordneten Objekten (25) aufweist.

6. Balg nach Anspruch 1, bei dem das Element (30) ein akustisches Filter ist.

7. Balg nach Anspruch 6, bei dem das akustische Filter ein Hochpass- oder ein Tiefpassfilter ist.

8. Balg nach Anspruch 1, bei dem das Element (40) eine akustische Linse ist.

9. Balg nach Anspruch 1, bei dem das Element (44) ein Absorber für akustische Wellen ist.

10. Balg nach einem der Ansprüche 1 bis 9, bei dem der Balg (2, 14, 23, 29, 39, 43) und das Element (9, 15, 16, 24, 30, 40, 44) aus dem gleichen Material ausgebildet sind.

11. Balg nach einem der Ansprüche 1 bis 8, bei dem das Element (9, 15, 16, 24, 30, 40) aus einem anderen Material als der Balg (2, 14, 23, 29, 39) ausgebildet ist, welches sich bezüglich seiner akustischen Eigenschaften von den akustischen Eigenschaften des akustischen Ausbreitungsmediums (8) unterscheidet.

12. Balg zur Ankopplung einer ein akustisches Ausbreitungsmedium (8) aufweisenden Quelle akustischer Wellen (1) an ein Lebewesen, wobei der Balg (12, 20, 26, 34, 41, 45) in dem Bereich (11), in dem er zur Einleitung von akustischen Wellen in das Lebewesen an dem Lebewesen zur Anlage gebracht werden kann, eine auf der dem Lebewesen abgewandten Seite des Balges (12, 20, 26, 34, 41, 45) definiert geometrisch veränderte Zone (13, 21, 22, 27, 35, 42, 46) aufweist, welche in dem Ausbreitungsweg (10) von mit der Quelle akustischer Wellen (1) erzeugten akustischen Wellen liegt und die akustischen Wellen gezielt formt.

13. Balg nach Anspruch 12, bei dem die Zone (13, 21, 22) des Balges (12, 20) derart ausgebildet ist, dass sie die Wirkung eines akustischen Spiegels entfaltet.

14. Balg nach Anspruch 13, bei dem die Zone (13, 21) in Form eines planen oder kegelförmigen akustischen Spiegels ausgebildet ist.

15. Balg nach Anspruch 13, bei dem die Zone (22) in Form eines wenigstens zwei geneigte Spiegelflächen aufweisenden akustischen Spiegels ausgebildet ist.

16. Balg nach Anspruch 12, bei dem die Zone (27) des Balges (26) eine Vielzahl von regelmäßig oder unregelmäßig angeordneten Objekten (28) aufweist.

17. Balg nach Anspruch 12, bei dem die Zone (35) des Balges (34) derart ausgebildet ist, dass sie die Wirkung eines akustischen Filters entfaltet.

18. Balg nach Anspruch 17, bei dem die Zone in Form eines akustischen Hochpass- oder eines akustischen Tiefpassfilters ausgebildet ist.

19. Balg nach Anspruch 12, bei dem die Zone (42) des Balges (41) derart ausgebildet ist, dass sie die Wirkung einer akustischen Linse entfaltet.

20. Balg nach Anspruch 12, bei dem die Zone (46) des Balges (45) derart ausgebildet ist, dass sie die Wirkung eines Absorbers für akustische Wellen entfaltet.

21. Balg zur Ankopplung einer ein akustisches Ausbreitungsmedium (8) aufweisenden Quelle akustischer Wellen (1) an ein Lebewesen, wobei der Balg (47, 49) in dem Bereich (11), in dem er zur Einleitung von akustischen Wellen in das Lebewesen an dem Lebewesen zur Anlage gebracht werden kann, einen Wandabschnitt (48, 51) aufweist, welcher aus einem anderen Material als der Rest des Balges (47, 49) ausgebildet ist, welcher Wandabschnitt (48, 51) in dem Ausbreitungsweg (10) von mit der Quelle akustischer Wellen (1) erzeugten akustischen Wellen liegt und die akustischen Wellen gezielt formt.

## Claims

1. A bellows containing an acoustic propagation medium (8) for coupling a source of acoustic waves (1) to a treatment subject, said bellows (2, 14, 23, 29, 39, 43) comprising, in the region (11) adapted to be seated against a treatment subject for introducing said acoustic waves into said treatment subject, a geometric element (9, 15, 16, 24, 30, 40, 44) firmly attached to said bellows (2, 14, 23, 29, 39, 43) in said region on a side of the bellows (2, 14, 23, 29, 39, 43) facing away from said treatment subject; and said geometrical element being disposed in a propagation path (10) of said acoustic waves (1) and shaping said acoustic waves in a designed manner.

2. A bellows as claimed in Claim 1, wherein said geometrical element (9, 15, 16) comprises an acoustic mirror.

3. A bellows as claimed in Claim 2, wherein said acoustic mirror (9, 15) is a planar mirror or conical mirror.

4. A bellows as claimed in Claim 2, wherein said acoustic mirror (16) comprises at least two inclined mirror faces (17, 18, 19).

5. A bellows as claimed in Claim 1, wherein said element (24) comprises a plurality of regularly or irregularly arranged objects (25).

6. A bellows as claimed in Claim 1, wherein said element (30) comprises an acoustic filter.

7. A bellows as claimed in Claim 6, wherein said acoustic filter is a highpass filter or a low-pass filter.

8. A bellows as claimed in Claim 1, wherein said element (40) comprises an acoustic lens.

9. A bellows as claimed in Claim 1, wherein said element (44) comprises an acoustic wave absorber.

10. A bellows as claimed in one of the Claims 1 to 9, with said bellows (2, 14, 23, 29, 39, 43) and said element (9, 15, 16, 24, 30, 40, 44) being comprised of the same material.

11. A bellows as claimed in one of Claims 1 to 8, with the element (9, 15, 16, 24, 30, 40) and the bellows (2, 14, 23, 29, 39) being comprised of different materials, each of said different materials having acoustic properties that differ from acoustic properties of said acoustic propagation medium (8).

12. A bellows for coupling a source of acoustic waves (1) containing an acoustic propagation medium (8) to a treatment subject, said bellows (12, 20, 26, 34, 41, 45) having, in the region adapted to be seated against a treatment subject for introducing said acoustic waves into said treatment subject, a defined geometrically changed zone (13, 21, 22, 27, 35, 42, 46) on the side of the bellows (12, 20, 26, 34, 41, 45) facing away from the subject which lies in a propagation path (10) of the acoustic waves (1) generated with the source of acoustic waves, said zone shaping said acoustic waves in a designed manner.

13. A bellows as claimed in Claim 12, with the zone (13, 21, 22) of the bellows (12, 20) being embodied such that it performs the function of an acoustic mirror.

14. A bellows as claimed in Claim 13, with the zone (13, 21) being embodied in the form of a planar or conical acoustic mirror.

15. A bellows as claimed in Claim 13, with the zone (22) being embodied in the form of an acoustic mirror comprising at least two inclined mirror faces.

16. A bellows as claimed in Claim 12, wherein the zone (27) comprises a plurality of regularly or irregularly arranged objects (28).

17. A bellows as claimed in Claim 12, wherein the zone (35) of the bellows is embodied such that it has the effect of an acoustic filter.

18. A bellows as claimed in Claim 17, wherein the zone is embodied in the form of an acoustic high-pass filter or an acoustic low-pass filter.

19. A bellows as claimed in claim 12, wherein the zone (42) is embodied such that it has the effect of an acoustic lens.

20. A bellows as claimed in Claim 12, wherein the zone (46) is embodied such that it has the effect of an acoustic wave absorber.

21. A bellows for coupling a source of acoustic waves (1), containing an acoustic propagation medium (8) to a treatment subject, said bellows (47, 49), in the region (11) adapted to be seated against a treatment subject for introducing said acoustic waves into said treatment subject, having a bellows wall section (48, 51) composed of a material that is different from the material of a remainder of the bellows (47, 51), said wall section (48, 51) being disposed in a propagation path (10) of acoustic waves generated by the source of the acoustic waves (1) and shaping said acoustic waves in a designed manner.

## Revendications

1. Soufflet pour coupler à un être vivant une source (1) d'ondes acoustiques ayant un milieu (8) de propagation acoustique, le soufflet (2, 14, 23, 29, 39, 43) comprenant, dans la région (11) dans laquelle il peut être appliqué contre l'être vivant pour introduire des ondes acoustiques dans l'être vivant, un élément (9, 15, 16, 24, 30, 40, 44) géométrique fixement assemblé au soufflet (2, 14, 23, 29, 39, 43) en étant disposé sur le côté du soufflet (2, 14, 23, 29, 39, 43) qui est opposé à l'être vivant, élément qui se situe dans la voie (10) de propagation des ondes acoustiques produites par la source (1) d'ondes acoustiques et qui met en forme les ondes acoustiques comme il convient.

2. Soufflet suivant la revendication 1, dans lequel l'élément (9, 15, 16) est un miroir acoustique.

3. Soufflet suivant la revendication 2, dans lequel le miroir (9, 15) acoustique est réalisé plan ou conique.

4. Soufflet suivant la revendication 2, dans lequel le miroir (16) acoustique comporte au moins deux faces (17, 18, 19) inclinées de miroir.

5. Soufflet suivant la revendication 1, dans lequel l'élément (24) comprend une pluralité d'objets (25) disposés régulièrement ou irrégulièrement.

6. Soufflet suivant la revendication 1, dans lequel l'élément (30) est un filtre acoustique.

7. Soufflet suivant la revendication 6, dans lequel le filtre acoustique est un filtre passe-haut ou passe-bas.

8. Soufflet suivant la revendication 1, dans lequel l'élément (40) est une lentille acoustique.

9. Soufflet suivant la revendication 1, dans lequel l'élément (44) est un absorbeur d'ondes acoustiques.

10. Soufflet suivant l'une des revendications 1 à 9, dans lequel le soufflet (2, 14, 23, 29, 39, 43) et l'élément (9, 15, 16, 24, 30, 40, 44) sont constitués du même matériau.

11. Soufflet suivant l'une des revendications 1 à 8, dans lequel l'élément (9, 15, 16, 24, 30, 40) est constitué d'un autre matériau que le soufflet (2, 14, 23, 29, 39), matériau qui possède des propriétés acoustiques différentes des propriétés acoustiques du milieu (8) de propagation acoustique.

12. Soufflet pour coupler à un être vivant une source (1) d'ondes acoustiques ayant un milieu (8) de propagation acoustique, le soufflet (12, 20, 26, 34, 41, 45) comprenant, dans la région (11) dans laquelle il peut être appliqué contre l'être vivant pour introduire des ondes acoustiques dans l'être vivant, une zone (13, 21, 22, 27, 35, 42, 46) géométriquement modifiée de manière définie sur le côté du soufflet (12, 20, 26, 34, 41, 45) qui est opposé à l'être vivant, zone qui se situe dans la voie (10) de propagation des ondes acoustiques produites par la source (1) d'ondes acoustiques et qui met en forme les ondes acoustiques de manière ciblée.

13. Soufflet suivant la revendication 12, dans lequel la zone (13, 21, 22) du soufflet (12, 20) est formée de telle sorte qu'elle agit comme un miroir acoustique.

14. Soufflet suivant la revendication 13, dans lequel la zone (13, 21) est réalisée sous la forme d'un miroir acoustique plan ou conique.

15. Soufflet suivant la revendication 13, dans lequel la zone (22) est réalisée sous la forme d'un miroir acoustique comportant au moins deux faces inclinées de miroir.

16. Soufflet suivant la revendication 12, dans lequel la zone (27) du soufflet (26) comprend une pluralité d'objets (28) disposés régulièrement ou irrégulièrement.

17. Soufflet suivant la revendication 12, dans lequel la zone (35) du soufflet (34) est formée de telle sorte qu'elle agit comme un filtre acoustique.

18. Soufflet suivant la revendication 17, dans lequel la zone est réalisée sous la forme d'un filtre acoustique passe-haut ou passe-bas.

19. Soufflet suivant la revendication 12, dans lequel la zone (42) du soufflet (41) est formée de telle sorte qu'elle agit comme une lentille acoustique.

20. Soufflet suivant la revendication 12, dans lequel la zone (46) du soufflet (45) est formée de telle sorte qu'elle agit comme un absorbeur d'ondes acoustiques.

21. Soufflet pour coupler à un être vivant une source (1) d'ondes acoustiques ayant un milieu (8) de propagation acoustique, le soufflet (47, 49) comprenant, dans la région (11) dans laquelle il peut être appliqué contre l'être vivant pour introduire des ondes acoustiques dans l'être vivant, un tronçon (48, 51) de paroi qui est constitué d'un autre matériau que le reste du soufflet (47, 49), le tronçon (48, 51) de paroi se situant dans la voie (10) de propagation des ondes acoustiques produites par la source (1) d'ondes acoustiques et mettant en forme les ondes acoustiques de manière ciblée.
